Europäisches Patentamt

European Patent Office

Office européen des brevets

(19)

(11) Publication number: 0 348 980
A2

## (12) EUROPEAN PATENT APPLICATION

(21) Application number: 89111877.0

(22) Date of filing: 29.06.89

(51) Int. Cl.⁴: C07C 43/12 , C07C 43/17 , C07C 19/08 , C07C 41/06 , C07C 17/26

(30) Priority: 30.06.88 IT 2116388

(43) Date of publication of application:
03.01.90 Bulletin 90/01

(84) Designated Contracting States:
DE ES FR GB NL SE

(71) Applicant: AUSIMONT S.r.l.
31, Foro Buonaparte
I-20100 Milano(IT)

(72) Inventor: Marraccini, Antonio
3, Corso Riviera
I-28040 Dormelletto Novara(IT)
Inventor: Pasquale, Antonio
8, Corso Milano
I-28100 Novara(IT)
Inventor: Vincenti, Marco
185, Corso Francia
I-10139 Torino(IT)

(74) Representative: Barz, Peter, Dr. et al
Patentanwälte Dr. V. Schmied-Kowarzik
Dipl.-Ing. G. Dannenberg Dr. P. Weinhold Dr.
D. Gudel Dipl.-Ing. S. Schubert Dr. P. Barz
Siegfriedstrasse 8
D-8000 München 40(DE)

(54) Telomers of 1,2-dichloro-1,2-difluoroethylene and process for preparing them.

(57) The present invention relates to telomers of 1,2-dichloro-1,2-difluoroethylene and to a process of preparing them.

1,2-dichloro-1,2-difluoroethylene is reacted, at a temperature of from -100°C to +50°C, with a perhalofluorooxy compound of formula

$$R_x-OF$$

wherein

$R_x$ represents, among other, a perhaloalkyl radical of from 1 to 10 carbon atoms.

Telomers are obtained which have, among others, the formulae

$$R_xO-(CFCl)_n-F$$
$$R_xO-(CFCl)_n-OR_x$$
$$R_y-(CFCl)_n-F \text{ and}$$
$$F-(CFCl)_n-F,$$

wherein

$R_y$ represents, among others, a perhaloalkyl radical containing from 1 to 9 carbon atoms.

Most of the resulting telomers are new products.

EP 0 348 980 A2

## TELOMERS OF 1,2-DICHLORO-1,2-DIFLUOROETHYLENE AND PROCESS FOR PREPARING THEM

The present invention relates to telomers of 1,2-dichloro-1,2-difluoroethylene.

More particularly, it relates to telomers obtained by reacting 1,2-dichloro-1,2-difluoroethylene with perhalofluorooxy compounds.

One object of the present invention is to provide a process which affords telomers of 1,2-dichloro-1,2-difluoroethylene which contain, besides end groups consisting of fluorine atoms, end groups of both the perhaloalkoxy, i.e., ether type (which, hence, are linked to the monomer units through an oxygen-carbon bond) and of the perhaloalkyl type (i.e., linked to the monomer units through a carbon-carbon bond).

Another object is the provision of a process capable of yielding telomers of 1,2-dichloro-1,2-difluoroethylene which contain a controllable, preferably high proportion of fluorine atoms as end groups.

A further object is to provide telomers in which the nature and the percent distribution of the end groups can be varied within a wide range.

Still a further object is to provide a process by which it is possible, within certain limits, to direct the telomerization reaction towards the formation of telomeric species having a low degree of telomerization or, on the contrary, a relatively high degree of telomerization.

Thus, the present invention is to provide a very flexible process, capable of affording a very wide range of products having different physical and chemical characteristics, which products can be employed in various fields of application.

These and further objects are achieved by the process for preparing telomers of 1,2-dichloro-1,2-difluoroethylene according to the present invention. This process is characterized in that 1,2-dichloro-1,2-difluorethylene is reacted, at a temperature of from about -100°C to about +50°C, with a perhalofluorooxy compound of formula

$R_x$-OF

wherein

$R_x$ represents a linear or branched perhalogenated alkyl, perhaloalkylmonoether or perhaloalkylpolyether radical of from 1 to 10 carbon atoms, which contains fluorine atoms or fluorine and chlorine atoms.

It is assumed that the reaction starts with the homolytic breakage of the O-F bond of the fluorooxy compound according to the scheme:

$R_x$-OF → $R_x$-O· + F·

resulting in $R_x$-O· and F· radicals which can act as initiators and terminators of telomerization.

Probably, the above $R_x$-O· radicals which contain more than one carbon atom are also capable of undergoing further fragmentation and rearrangement reactions with other radical species being formed which, in turn, can also act as telomerization initiators and terminators.

Hence, by starting from a perhalofluorooxy compound, one can form radicals $R^1$ derived from radicals $R_x$, in which $R_x$ of at least two carbon atoms has lost at least one carbon atom and/or $R_x$ of at least 3 carbon atoms has undergone a rearrangement. More commonly, the radical $R^1$ is a radical $R_y$ containing a lower number of carbon atoms than $R_x$. Thus, when $R_x$ contains from 2 to 10 carbon atoms, $R_y$ contains from 1 to 9 carbon atoms.

Furthermore, during the chain growth reaction, the teleomeric radicals are presumably capable of undergoing exchange reactions with other radicals with, for example, C1 radicals being formed which, in turn, can act as telomerization initiators and terminators. The above exchange reactions can be particularly promoted when the reaction is carried out in the presence of $F_2$, according to the procedure described below.

Rearrangement reactions of the telomeric radicals can also occur with terminal double bonds being formed according to the scheme:

-CFCl-CFCl· → -CF=CFCl + Cl·

It has been found according to the present invention that the telomerization products are composed of a mixture of telomers having different end groups and different degrees of telomerization.

When the reaction is carried out in the absence of fluorine, the telomers obtained are predominantly the following:

| | |
|---|---|
| $R_xO\text{-}(CFCl)_n\text{-}F$ | (A) |
| $R_xO\text{-}(CFCl)_n\text{-}OR_x$ | (B) |
| $R_y\text{-}(CFCl)_n\text{-}F$ | (C) |
| $F\text{-}(CFCl)_n\text{-}F$ | (D) |

wherein:

n ranges from 4 to 20;

$R_x$ is defined as specified above;

$R_y$ is a radical derived from $R_x$, wherein $R_x$ containing at least two carbon atoms has lost at least one carbon atom; therefore, $R_y$ is a linear or branched perhalogenated alkyl radical, a perhaloalkylomonether radical or a perhaloalkylpolyether radical of from 1 to 9 carbon atoms, which contains fluorine atoms or fluorine and chlorine atoms.

The telomers (A), (B) and (C) are new products.

In addition to the above, small amounts of the following telomers, characterized by an unsaturation in chain-end position can also be present.

| | |
|---|---|
| $R_xO\text{-}(CFCl)_{n\text{-}2}\text{-}CF = CFCl$ | (Q) |
| and | |
| $F\text{-}(CFCl)_{n\text{-}2}\text{-}CF = CFCl$ | (R) |

The telomers (Q) are also new products.

When the reaction is carried out in the presence of fluorine and at rather high temperatures under conditions as defined below the following telomers are also formed:

| | |
|---|---|
| $R_xO\text{-}(CFCl)_n\text{-}Cl$ | (E) |
| $F\text{-}(CFCl)_n\text{-}Cl$ | (F) |
| $Cl\text{-}(CFCl)_n\text{-}Cl$ | (G) |
| $F\text{-}[(CFCl)_{n\text{-}1}CF_2]\text{-}F$ | (H) |
| $F\text{-}[(CFCl)_{n\text{-}1}CF_2]\text{-}OR_x$ | (I) |
| $R_xO\text{-}[(CFCl)_{n\text{-}m}(CF_2)_m]\text{-}OR_x$ | (L) |
| $R_xO\text{-}[(CFCl)_{n\text{-}m}(CF_2)_m]\text{-}Cl$ | (M) |
| $R_y\text{-}[(CFCl)_{n\text{-}m}(CF_2)_m]\text{-}Cl$ | (N) |
| $F\text{-}[(CFCl)_{n\text{-}m}(CF_2)_m]\text{-}Cl$ | (O) |
| $Cl\text{-}[(CFCl)_{n\text{-}m}(CF_2)_m]\text{-}Cl$ | (P) |

wherein m is 1 or 2.

In the telomers (H), (I), (L), (M), (N), (O) and (P) the $CF_2$ groups can be in any position along the chain of monomer units.

The telomers (E), (I), (L), (M) and (N) are new products.

In the telomer mixtures obtained, both in the presence and in the absence of fluorine, small amounts of telomers, having formulae different from the ones shown above may also be present.

When $R_x$ and $R_y$ are perhaloalkylpolyether radicals, they preferably contain two oxygen atoms.

The radicals $R_x$ and $R_y$ preferably are perfluorinated radicals.

Preferably, $R_x$ contains from 1 to 6 carbon atoms while $R_y$ contains from 1 to 5 carbon atoms.

When $R_x$ and $R_y$ contain chlorine atoms, they preferably do not contain $-CCl_3$ groups.

In the preferred products n generally ranges from 4 to 10.

In order to carry out the reaction, generally an either gaseous or liquid stream of perhalofluorooxy compound is introduced into a reactor which contains 1,2-dichloro-1,2-difluorethylene either in the liquid state or dissolved in a suitable solvent. Since the boiling point of 1,2-dichloro-1,2-difluorethylene is about $+22°$ C, the reaction should suitably be carried out below said temperature. In case pressures higher than atmospheric pressure (under which 1,2-dichloro-1,2-difluorethylene can be kept in the liquid state) are used, the reaction can be carried out at temperatures higher than $+22°$ C.

If the reaction is to be carried out in solution, a solvent for 1,2-dichloro-1,2-difluorethylene, which is inert under the reaction conditions, can be used. In particular, a chlorofluorocarbon such as, for example,

EP 0 348 980 A2

1,2-dichlorotetrafluoroethane, fluorotrichloromethane and dichlorodifluoromethane may be employed for this purpose. The concentration of 1,2-dichloro-1,2-difluoroethylene in the solution generally ranges from 20 to 95% by weight.

Generally, the fluorooxy compound is fed to the reactor in the form of a gaseous stream. The gaseous fluorooxy compound preferably is diluted with a gas inert under the reaction conditions. Examples of inert diluent gases are nitrogen, argon, helium and gaseous chlorofluorocarbons, selected, for example, from 1,2-dichlorotetrafluoroethane, dichlorodifluoromethane and mixtures thereof. When the fluorooxy compound is $CF_3OF$, it can be fed in the absence of an inert gas or in the presence of any amount of an inert gas, i.e., $CF_3OF$ can be used at a concentration of from 0.01% to 100% by volume, based on the mixture of $CF_3OF$ and inert gas.

When the reaction is carried out with fluorooxy compounds containing at least two carbon atoms, the concentration of the fluorooxy compound in the mixture of said fluorooxy compound and inert gas generally is from 0.05% to 25% by volume.

When the reaction is carried out in the presence of fluorine, the amount of said fluorine generally ranges from 10 to 90 parts by volume per 100 parts of mixture of fluorine and fluorooxy compound. Smaller amounts of fluorine can also be used (for example, from 1 to 10 parts by volume per 100 parts of mixture of fluorine and fluorooxy compound) but under such conditions the influence of fluorine on the nature of the terminal groups obtained is less pronounced.

If the fluorooxy compound is fed to the reactor in the liquid state, it is mixed with a liquid inert under the reaction conditions, in particular a chlorofluorocarbon, for example 1,2-dichlorotetrafluoroethane, fluorotrichloromethane and dichlorodifluoromethane (or mixtures thereof) and is carried, in aerosol form, by a gas inert under the reaction conditions, for example, nitrogen, argon or helium.

To the reactor containing a solvent for 1,2-dichloro-1,2-difluoroethylene a stream of fluorooxy compound in the gaseous or liquid state can be fed according to any of the feeding procedures outlined above, with a stream of 1,2-dichloro-1,2-difluoroethylene in gaseous or liquid form being introduced simultaneously. In this case, 1,2-dichloro-1,2-difluoroethylene is preferably employed in the liquid state.

Of course, other procedures can be used in order to bring the reactants into contact with each other. For example, liquid 1,2-dichloro-1,2-difluoroethylene can be fed to a reactor containing the fluorooxy compound dissolved in a suitable solvent, in particular dissolved in a chlorofluorocarbon selected from, for example, 1,2-dichlorotetrafluoroethane, fluorotrichloromethane, dichlorodifluoromethane and mixtures thereof.

The temperature at which the reaction is carried out ranges from about -100°C to about +50°C. For each fluorooxy compound, the reaction should be carried out at a temperature which is at least equal to the threshold temperature of its reaction with 1,2-dichloro-1,2-difluoroethylene. Said threshold temperature is different for each telogen. Preferably, the reaction is carried out at a temperature within the range of from -100°C to -20°C.

The reaction can be directed so that is predominantly yields the telomeric species (A), (B), (C) and (D). This result can be obtained by operating in the absence of elemental fluorine or in the presence of small amounts thereof (for example, within the range of from 1 to 3% by volume in the mixture of fluorooxy compound and $F_2$) and, simultaneously, under such conditions as to decrease the amount of heat development inside the reaction medium.

These conditions are:

(a) An effective stirring of the reaction medium;

(b) Use of reaction temperatures within a relatively low range, provided that they are still higher than the threshold reaction temperature, i.e., in particular, use of temperatures of from -100 up to -60°C;

(c) Reduction of the telogen flowrate;

(d) Increasing the telogen dilution.

In order to obtain the above result, condition (a) is coupled with one or more (all) of conditions (b), (c) and (d).

By operating according to the above procedure, said telomers (A), (B), (C) and (D) can be obtained in proportions which may reach, or even exceed, 90% by weight of the total of reaction products.

The reaction can also be directed towards the formation of relatively large amounts of telomers (C), i.e., of those containing an end group of the perhaloalkyl type, by operating with less intense stirring and at higher temperatures of the reaction medium (in particular within the range of from -40 to -60°C).

The same result can be obtained by using a higher flowrate of the telogen, at lower temperatures (for example, of from -60 to -80°C) and with a lower degree of dilution of the telogen in the inert gas.

By operating in the presence of large amounts of elemental fluorine (within the range of from 10 to 50 parts by volume of $F_2$ per 100 parts of mixture of $F_2$ and the fluorooxy compound) and at relatively high

4

temperatures (within the range of from -60°C to about -20°C), the proportion of telomers containing end groups -F and -Cl, in particular of the species (D), (F), (G), (O) and (P), can be increased.

By operating in the presence of large amounts of elemental fluorine (within the range of from 25 to 95 parts by volume of $F_2$ per 100 parts of mixture of $F_2$ and fluorooxy compound), at lower temperatures (within the range of from -100°C up to about -60°C) and with high degree of dilution of fluorooxy compound and fluorine in the carrier gas, a high proportion of telomers may be obtained wherein one or both end groups are -F. The expression "high degree of dilution of fluorooxy compound" is meant to indicate, for example, 1 to 3 parts by volume of the total amount of fluorooxy compound and elemental fluorine per 100 parts of diluent gas. By operating according to these modalities, in particular the telomers (A), (B) and (D) with a large proportion of the (D) species can be obtained.

According to a further aspect of the process of the present invention, when adopting the conditions under which the telomerization reaction can be directed towards the prevailing formation of the desired telomeric species, the degree of telomerization can, furthermore, be directed, within certain limits, towards low values. Said result can be achieved by using one or more (or all) of the following conditions:
- Low telogen flowrates;
- Relatively high degree of dilution of the telogens;
- Low reaction temperature;
- Use of 1,2-dichloro-1,2-difluoroethylene in the presence of a solvent thereof.

For example, by adopting the above conditions, more than 95% of the telomers having values of n within the range of from 4 to 6 can be obtained.

The products obtainable by the process according to the present invention can be used as electrical insulators, lubricants and heat transfer media.

They are, furthermore, intermediate products for the preparation, by de-chlorination, of olefinic products, for example, perfluorobutadiene, disclosed in copending European patent application, case No. D3814 (which is being filed on the same day as the instant one) by applicant. These olefinic products are useful for preparing polymeric products, dielectric fluids and heat exchange fluids. In these applications, the end products obtained by means of the process according to the present invention can be used as obtained, i.e., as mixtures or, after an enrichment - by distillation - with certain components, for example, with components having a low degree of telomerization. Furthermore, as already explained, the use of particular operating conditions makes it possible to obtain mixtures which contain relatively large amounts of one or more specific telomeric species.

The presence of a considerable amount of determined end groups (perhaloalkoxy, perhaloalkyl, -Cl, -F) or of a prevailing amount of -F end groups as well as the prevalence of a relatively low or relatively high degree of telomerization and the possibility of varying the telogen employed as starting material afford various ranges of products with particular physico-chemical properties. Said properties make it possible to employ the telomeric products in various fields of application.

Examples of specific fluorooxy compounds which can be used in the process of the present invention include:

fluorooxy trifluoromethane;
fluorooxy pentafluoroethane;
1-fluorooxy heptafluoropropane;
1-fluorooxy nonafluorobutane;
1-fluoro-2-chlorotetrafluoroethane;
1-fluorooxy-2,2'-dichlorotrifluoroethane;
fluorooxy heptafluoroisopropane;
fluorooxy nonafluoroisobutane;
fluorooxy nonafluoro-tert.-butane;
1-fluorooxy-2-perfluoro-n-propoxy-hexafluoropropane;
1-fluorooxy-2-perfluoromethoxy-hexafluoropropane;
1-fluorooxy-2-perfluoroethoxy-hexafluoropropane and
1-fluorooxy-3-chloro-hexafluoropropane.

When $CF_3OF$ is used as telogen in the absence or in the presence of small amounts of $F_2$ (up to 3% by volume in the mixture of $CF_3OF$ and $F_2$) and of an inert diluent (up to 33% by volume of $CF_3OF$ in the mixture of $CF_3OF$ and $N_2$), the mixture of telomers obtained is almost exclusively composed of the following species:

| | |
|---|---|
| $CF_3O\text{-}(CFCl)_n\text{-}F$ | (Ia) |
| $CF_3O\text{-}(CFCl)_n\text{-}OCF_3$ | (IIa) |
| $F(CFCl)_n\text{-}F$ | (IIIa) |

(Ia) and (IIa) are new products.

(Ia) and (IIa) may represent more than 90% of the reaction products. Products with values of n of from 4 to 6 can constitute more than 99% of the crude reaction product.

By employing $CF_3OF$ or $CF_3OF$ and $F_2$ (according to volume ratios of from 1:1 to 1:3) with high degrees of dilution in an inert gas (for example, with a concentration of $CF_3OF$ of about 1%), the species (IIIa) may represent up to 90% of the above species.

When $CF_3OF$ is used as telogen in the presence of large amounts of $F_2$ (for example, 70% by volume in the mixture of $CF_3OF$ nd $F_2$) and with low degrees of dilution in an inert diluent gas, at relatively high temperatures (for example about -40° C), besides the above telomers, the following species are also formed:

| | |
|---|---|
| $F\text{-}(CFCl)_n\text{-}Cl$ | (IVa) |
| $Cl\text{-}(CFCl)_n\text{-}Cl$ | (Va) |
| $CF_3O\text{-}(CFCl)_n\text{-}Cl$ | (VIa) |
| $F\text{-}[(CFCl)_{n\text{-}1}CF_2]\text{-}F$ | (VIIa) |
| $F\text{-}[(CFCl)_{n\text{-}1}CF_2]\text{-}OCF_3$ | (VIIIa) |
| $CF_3O\text{-}[(CFCl)_{n\text{-}1}CF_2]\text{-}OCF_3$ | (IXa) |
| $F\text{-}[(CFCL)_nCF_2]\text{-}Cl$ | (Xa) |
| $CF_3O\text{-}[(CFCL)_nCF_2]\text{-}Cl$ | (XIa) |
| $Cl\text{-}[(CFCL)_nCF_2]\text{-}Cl$ | (XIIa) |

(VIa), (VIIIa), (IXa) and (XIa) are new species.

Under the conditions just mentioned the species having initial F and Cl atoms and terminal F and Cl atoms are predominant and can constitute up to and even more than 90% of the telomer mixture obtained. Under these conditions species with values of n equal to 4 can represent up to or even more than 75% of the mixture.

By employing $CF_3OF$ as the telogen and using or not using large amounts of fluorine, small amounts of other species may be formed, such, as, for example:

| | |
|---|---|
| $F\text{-}(CFCl)_{n\text{-}2}\text{-}CF = CFCl$ | (IIIb) |
| $CF_3O\text{-}(CFCl)_{n\text{-}2}\text{-}CF = CFCl$ | (Ib) |
| $CF_3O\text{-}[(CFCl)_{n\text{-}1}\text{-}CFH]\text{-}F$ | (I′b) |

(Ib) and (I′b) are new species. Species (IIIb) and (Ib) can be formed through the loss of a Cl· radical from the radical chain. Species (I′b) can be formed by substitution of an H atom for a Cl atom in case hydrogen-containing substances (for example, small amounts of water) are present in the reaction medium.

In the species (I′b), the -CFH- group can be in any position along the chain of monomer units.

When $CF_3CF_2OF$ is used as the telogen in the absence, or in the presence of small amounts, of $F_2$ (up to 17% by volume in the mixture of $CF_3CF_2OF$ and $F_2$), the mixture of telomers obtained is prevailingly composed of the following species:

| | |
|---|---|
| $CF_3CF_2O\text{-}(CFCl)_n\text{-}F$ | (Ic) |
| $CF_3CF_2O\text{-}(CFCl)_n\text{-}OCF_2CF_3$ | (IIc) |
| $CF_3\text{-}(CFCl)_n\text{-}F$ | (IIIc) |
| $F\text{-}(CFCl)_n\text{-}F$ | (IIIa) |
| $CF_3\text{-}(CFCl)_n\text{-}OCF_2CF_3$ | (IVc) |
| $CF_3CF_2O\text{-}(CFCl)_{n\text{-}2}\text{-}CF = CFCl$ | (I′c) |

Telomers (Ic), (IIc), (IIIc), (IVc) and (I′c) are new products.

6

When $CF_3$-$CF_2$-$CF_2$-OF is used as the telogen at low temperatures (within the range of from -90°C up to -75°C) and in the absence, or in the presence of small amounts, of $F_2$ (up to 10% by volume in the mixture of $CF_3$-$CF_2$-$CF_2$-OF and $F_2$), the mixture of telomers obtained is prevailingly composed of the following species (more than 95% of the species present):

| | |
|---|---|
| $CF_3CF_2CF_2O$-$(CFCl)_n$-$F$ | (Id) |
| $CF_3CF_2CF_2O$-$(CFCl)_n$-$OCF_2CF_2CF_3$ | (IId) |
| $CF_3CF_2$-$(CFCl)_n$-$OCF_2CF_2CF_3$ | (IIId) |
| $F$-$(CFCl)_n$-$F$ | (IIIa) |

When $CF_3$-$CF_2$-$CF_2$-OF is used as the telogen, at higher temperatures (within the range of from -40°C to -65°C), in the absence, or in the presence of small amounts, of $F_2$, employing high concentrations (for example, 50%) of 1,2-dichloro-2,3-difluoroethylene in a solvent, the mixture obtained is predominantly composed of the following species:

| | |
|---|---|
| $CF_3CF_2CF_2O$-$(CFCl)_n$-$F$ | (Id) |
| $CF_3CF_2CF_2O$-$(CFCl)_n$-$OCF_2CF_2CF_3$ | (IId) |
| $CF_3CF_2$-$(CFCl)_n$-$OCF_2CF_2CF_3$ | (IIId) |
| $F$-$(CFCl)_n$-$F$ | (IIIa) |
| $CF_3O$-$(CFCl)_n$-$F$ | (Ia) |
| $CF_3O$-$(CFCl)_n$-$OCF_2CF_2CF_3$ | (IVd) |
| $CF_3CF_2CF_2O$-$[(CFCl)_{n-m}(CF_2)_m]$-$F$ | (Vd) |
| $CF_3CF_2CF_2O$-$CF_2CF_2CF_2O$-$(CFCl)_n$-$F$ | (VId) |
| $CF_3CF_2CF_2O$-$(CFCl)_n$-$Cl$ | (VIId) |
| $F$-$[(CFCl)_{n-m}(CF_2)_m]$-$F$ | (VIIId) |
| $CF_3O$-$[(CFCl)_{n-m}(CF_2)_m]$-$F$ | (IXd) |
| $CF_3O$-$(CFCl)_n$-$OCF_3$ | (IIa) |
| $CF_3CF_2CF_2O$-$[(CFCl)_{n-m}(CF_2)_m]$-$Cl$ | (Xd) |
| $CF_3CF_2CF_2O$-$[(CFCl)_{n-1}(CFH)]$-$F$ | (XId) |
| $F$-$(CFCl)_{n-2}$-$CF = CFCl$ | (IIIb) |
| $CF_3O$-$(CFCl)_{n-2}$-$CF = CFCl$ | (Ib) |
| $CF_3CF_2CF_2O$-$(CFCl)_{n-2}$-$CF = CFCl$ | (Ie) |

wherein m is 1 or 2.

(Id), (IId), (IIId), (Ia), (IVd), (Vd), (VId), (VIId), (IXd), (IIa), (Xd), (XId), (Ib) and (Ie) are new species.

(Id), (IId), (IIIa), (Ia) and (IVd) may represent up to or even more than 70% of the species present.

The main advantages of the process according to the present invention can be summarized as follows:

1. The percent distribution and the nature of the end groups present in the telomeric species obtained can be varied within a wide range and, within certain limits, the degree of telomerization can also be varied. Thus, a very wide range of products having different physical and chemical properties can be obtained which properties are suitable for meeting a host of requirements in many fields of application.

2. When the reaction is carried out in a way such as to result in no or at least very few -Cl end groups, the resulting telomers are endowed with high chemical and thermal stabilities.

The following examples are given in order to illustrate the invention without, however, limiting it.

## EXAMPLE 1

A stream of 1.0 Nl/hour of $CF_3OF$ and 2.0 Nl/hour of $N_2$ was continuously bubbled through 178 g of liquid 1,2-dichloro-1,2-difluoroethylene (DCDFE), cooled at -40°C, in a reactor equipped with condenser, thermometer and stirrer.

After six hours the reaction was stopped, unreacted DCDPE was distilled off and 45.1 g of product were recovered.

The product was analyzed by gas-chromatography on a 3% SP-2100 column and by gas-mass and $^{18}$F-N.M.R. spectroscopy.

The product was composed of the telomeric species (Ia) to (IXa). The species (Ia) and (IIa) represented more than 90% of the mixture. Species (IIIa) accounted for about 4% of the mixture.

From the gas-chromatographic analysis it was concluded that species having values of within the range of from 4 to 6 constituted about 96% of the crude reaction mixture.

EXAMPLE 2

Following the procedure of example 1, 1.5 Nl/hour of $CF_3OF$ and 3 NL/hour of $N_2$ were bubbled (for 9 hours) through 178.3 g of DCDFE mixed with 43.7 g of 1,2-dichlorotetrafluoroethane, kept at a temperature of -75° C.

After removal of unreacted DCDFE and solvent, 24.4 g of product were obtained.

The product obtained contained (Ia), (IIa) and (IIIa) as well as traces of

$CF_3O\text{-}(CFCl)_2\text{-}CF = CFCl$.

Species (Ia) and (IIa) represented more than 95% of the mixture while the species (IIIa) accounted for about 2% of the reaction product.

Species with n values within the range of from 4 to 6 constituted more than 99% of the crude reaction product.

EXAMPLE 3

Following the procedure of example 1, 1.5 Nl/hour of $CF_3OF$ and 3 Nl/hour of $N_2$ were bubbled (for 6.5 hours) through 170 g of DCDFE kept at -5° C.

After removing the unreacted DCDFE, 20.5 g of product were obtained.

The product obtained contained the species (Ia), (IIa) and (IIIa)

| | |
|---|---|
| $CF_3O\text{-}(CFCl)_{n-2}\text{-}CF = CFCl$ | (Ib) |
| and | |
| $F\text{-}(CFCl)_{n-2}\text{-}CF = CFCl$ | (IIIb) |

Species (Ia) and (IIa) constituted more than 85% of the mixture, while species (IIIa) constituted about 12% thereof.

Species with n values comprised within the range of from 3 to 6 represented more than 99% of the crude product.

EXAMPLE 4

Following the procedure of example 1, 0.3 Nl/hour of $CF_3OF$, 0.7 Nl/hour of fluorine and 2 Nl/hour of $N_2$ were bubbled (for 6 hours) through 151 g of DCDFE kept at -40° C.

After removing the unreacted DCDFE, 12 g of a reaction product composed of species (Ia) to (XIIa) were recovered.

Species (IIIa), (IVa), (Va), (VIIa), (Xa) and (XIIa) constituted more than 00% of the mixture. Species (IIIa) accounted for about 27% of said mixture.

Species with n values of 4 represented about 75% of the crude product.

EXAMPLE 5

Following the procedure of example 1, 1 Nl/hour of $CF_3CF_2OF$ and 5 Nl/hour of $N_2$ were bubbled through 190 g of 1,2-dichlorotetrafluoroethane containing 10 g of DCDFE, kept at -75° C.

During the whole reaction time of 5 hours additional 28 g of DCDFE were successively added.

After removing the solvent and the unreacted olefin, species (Ic) to (IVc), (I'c) and (IIIa) were present in the crude product.

Among these, (IIIa) accounted for about 20% and (Ic) and (IIc) represented about 70% of the product.

EP 0 348 980 A2

Species with n values within the range of from 4 to 6 constituted about 93% of the product.


EXAMPLE 6

Following the procedure of example 1, 0.8 Nl/hour of $CF_3CF_2CF_2OF$, 0.2 Nl/hour of $F_2$ and 2.4 Nl/hour of $N_2$, pre-cooled at -40°C, were bubbled through 45 g of DCDFE, dissolved in 300 g of 1,2-dichlorotetrafluoroethane kept at -75°C.

During the bubbling, additional 63 g of DCDFE were successively added. The total reaction time was 17.5 hours.

After removing the solvent and the unreacted DCDFE, the telomeric product was recovered. Said product was composed of species (Id), (IId), (IIId) and (IIIa).

Species (Id), (IId) and (IIId) represented more than 70% of the mixture while species (IIIa) accounted for about 27%.

Species with n values of 4 constituted about 90% of the crude product.


EXAMPLE 7

Following the procedure of example 6, 1.2 Nl/hour of $CF_3CF_2CF_2OF$, 0.1 Nl/hour of F2 and 5 Nl/hour of $N_2$ were cooled to -45°C and bubbled through 30 g of DCDFE dissolved in 500 g of $CF_2Cl_2$, kept at -92°C.

During the reaction 43.5 g of DCDFE were successively added. The total reaction time was 6.5 hours.

After removing the solvent and the unreacted olefin, telomers (Ia), (IIa) and (IIIa), of which the species (IIIa) accounted for 45%, were present.

The species with n values of 4 constituted about 95% of the product.


EXAMPLE 8

Following the procedure of example 6, 1 Nl/hour of $CF_3CF_2CF_2OF$ and 4 Nl/hour of $N_2$, cooled to -25°C, were bubbled through 110 g of 1,2-dichlorotetrafluoroethane containing 90 g of DCDFE and kept at -65°C.

The total reaction time was 7 hours.

After removal of the solvent and the unreacted olefin, the species (Id), (IId), (IIId), (IIIa), (Ia), (IVd), (Vd), (VId), (VIId), (VIIId), (IXd), (IIa), (Xd), (XId), (IIIb), (Ib) and (Ie) were present in the crude reaction product. Species (IIIa) accounted for about 24% of the product.

Species with n values of 4 constituted about 70% of the product.


EXAMPLE 9

In a reactor equipped with condenser, thermometer and stirrer, a gas stream consisting of 0.6 Nl/hour of $F_2$ and 103 Nl/hour of $N_2$ was continuously bubbled through 151 g of liquid DCDFE kept at -72°C.

After 6 hours the reaction was stopped and unreacted DCDFE was distilled off, leaving 37.33 g of product.

Said product was analysed by gas-chromatography on a 3% SP 2100 packed column, by gas mass and, finally by $^{19}$F-N.M.R. spectroscopy.

The product was composed of telomers (Ia) to (IIIa).

The species (Ia) and (IIa) represented 13% and the species (IIIa) constituted about 87% of the mixture.

From the gas-chromatographic analysis it was concluded that species with values of n within the range of from 4 to 6 constituted 100% of the crude product.


EXAMPLE 10

In a reactor equipped with condenser, thermometer and stirrer, a gas stream consisting of 1.2 Nl/hour of fluorooxy trifluoromethane and 103 Nl/hour of $N_2$ was continuously bubbled through 150 g of liquid DCDFE kept at -72°C.

After 6 hours the reaction was stopped and unreacted DCDFE was distilled off, leaving 45 g of product.

Said product was analysed by gas-chromatography on a 3% SP 2100 packed column, by gas mass and, finally by $^{19}$F-N.M.R. spectroscopy.

The product was composed of telomers (Ia) to (IXa).

The species (Ia) and (IIa) accounted for about 38% of the mixture while species (IIIa) represented about 58% of the mixture.

From the gas-chromatographic analysis it was concluded that species with values of n within the range of from 4 to 6 represented about 97% of the crude product.

## EXAMPLE 11

In a reactor equipped with condenser, thermometer and stirrer a gas stream consisting of 1.2 Nl/hour of fluorooxytrifluoromethane and 103 Nl/hour of $N_2$ was continuously bubbled through 150 g of a liquid mixture consisting of 75% by weight of DCDFE and 25% of trichlorotrifluoroethane kept at -72°C.

After 6 hours the reaction was stopped and unreacted DCDFE was distilled off, leaving 28.15 g of a mixture of telomers.

The product was analysed by gas-chromatography on a 3% SP 2100 packed column, by gas mass and, finally, by $^{19}$F-N.M.R. spectroscopy.

The product was composed of telomers (Ia), (IIa) and (IIIa).

Telomers (Ia) and (IIa) constituted about 17% of the mixture. Species (IIIa) accounted for about 83% of the mixture.

From the gas-chromatographic analysis it was concluded that species with n values of 4 constituted about 98% of the crude product.

## Claims

1. Process for preparing telomers of 1,2-dichloro-1,2-difluoroethylene, characterized in that 1,2-dichloro-1,2-difluoroethylene is reacted, at a temperature of from about -100°C to about +50°C, with a perhalofluorooxy compound of formula

$R_x$-OF

wherein

$R_x$ represents a linear or branched perhaloalkyl, perhaloalkylmonoether or perhaloalkylpolyether radical of from 1 to 10 carbon atoms, which contains fluorine atoms or fluorine and chlorine atoms.

2. Process according to claim 1, characterized in that, when $R_x$ is a perhaloalkylpolyether radical, it contains two oxygen atoms.

3. Process according to claim 1 or 2, characterized in that $R_x$ is a perfluorinated radical or that, when $R_x$ contains chlorine atoms, it does not comprise -CCl$_3$ groups.

4. Process according to one or more of the preceding claims, characterized in that $R_x$ contains from 1 to 6 carbon atoms.

5. Process according to one or more of the preceding claims, characterized in that a gaseous stream of a perhalofluorooxy compound is introduced into a reactor which contains 1,2-dichloro-1,2-difluoroethylene, either in the liquid state or dissolved in a solvent inert under the reaction conditions, preferably in a chlorofluorocarbon.

6. Process according to one or more of the preceding claims, characterized in that, before being introduced, the perhalofluorooxy compound is diluted with a gas inert under the reaction conditions, preferably nitrogen, argon, helium or a chlorofluorocarbon gas.

7. Process according to one or more of claims 1 to 4, characterized in that the perhalofluorooxy compound is introduced in the liquid state into a reactor which contains 1,2-dichloro-1,2-difluoroethylene, either in the liquid state or dissolved in a solvent inert under the reaction conditions, with the perhalofluorooxy compound being mixed with a liquid inert under the reaction conditions, or being carried as an aerosol, by a gas inert under the reaction conditions, preferably nitrogen, argon or helium.

8. Process according to claim 7, characterized in that the liquid inert under the reaction conditions is a chlorofluorocarbon.

9. Process according to one or more of the preceding claims, characterized in that fluorine is fed to the reaction medium in an amount of from 1 to 90 parts by volume, preferably 10 to 90 parts by volume, per 100 parts of mixture of fluorine and perhalofluorooxy compound.

10. Process according to one or more of the preceding claims, characterized in that the reaction is carried out at a temperature of from about -100°C to about -20°C.

11. Telomers of 1,2-dichloro-1,2-difluoroethylene having the formulae:

| | |
|---|---|
| $R_xO\text{-}(CFCl)_n\text{-}F$ | (A) |
| $R_xO\text{-}(CFCl)_n\text{-}OR_x$ | (B) |
| $R_y\text{-}(CFCl)_n\text{-}F$ | (C) |
| $R_xO\text{-}(CFCl)_{n\text{-}2}\text{-}CF = CFCl$ | (Q) |
| $R_xO\text{-}(CFCl)_n\text{-}Cl$ | (E) |
| $F\text{-}[(CFCl)_{n\text{-}1}CF_2]\text{-}OR_x$ | (I) |
| $R_xO\text{-}[(CFCl)_{n\text{-}m}(CF_2)_m]\text{-}OR_x$ | (L) |
| $R_xO\text{-}[(CFCl)_{n\text{-}m}(CF_2)_m]\text{-}Cl$ | (M) |
| $R_y\text{-}[(CFCl)_{n\text{-}m}(CF_2)_m]\text{-}Cl$ | (N) |

and mixtures thereof, wherein
$R_x$ is a linear or branched perhaloalkyl, perhaloalkylmonoether or perhaloalkylpolyether radical of from 1 to 10 carbon atoms, which contains fluorine atoms or fluorine and chlorine atoms;
$R_y$ is a linear or branched perhaloalkyl, perhaloalkylmonoether or perhaloalkylpolyether radical of from 1 to 9 carbon atoms, which contains fluorine atoms or fluorine and chlorine atoms;
n ranges from 4 to 20, preferably from 4 to 10; and m is 1 or 2.

12. Mixtures of telomers of 1,2-dichloro-1,2-difluoroethylene, prevailingly containing species of the formulae

| | |
|---|---|
| $R_xO\text{-}(CFCl)_n\text{-}F$ | (A) |
| $R_xO\text{-}(CFCl)_n\text{-}OR_x$ | (B) |
| $R_y\text{-}(CFCl)_n\text{-}F$ | (C) |
| $F\text{-}(CFCl)_n\text{-}F$ | (D) |

wherein $R_x$, $R_y$ and n are defined as in claim 11.

13. Mixtures of telomers of 1,2-dichloro-1,2-difluoroethylene containing, in addition to the species of formulae (A), (B), (C) and (D), some or all of the species of the following formulae:

| | |
|---|---|
| $F\text{-}(CFCl)_n\text{-}F$ | (D) |
| $R_xO\text{-}(CFCl)_n\text{-}Cl$ | (E) |
| $F\text{-}(CFCl)_n\text{-}Cl$ | (F) |
| $Cl\text{-}(CFCl)_n\text{-}Cl$ | (G) |
| $F\text{-}[(CFCl)_{n\text{-}1}CF_2]\text{-}F$ | (H) |
| $F\text{-}[(CFCl)_{n\text{-}1}CF_2]\text{-}OR_x$ | (I) |
| $R_xO\text{-}[(CFCl)_{n\text{-}m}(CF_2)_m]\text{-}OR_x$ | (L) |
| $R_xO\text{-}[(CFCl)_{n\text{-}m}(CF_2)_m]\text{-}Cl$ | (M) |
| $R_y\text{-}[(CFCl)_{n\text{-}m}(CF_2)_m]\text{-}Cl$ | (N) |
| $F\text{-}[(CFCl)_{n\text{-}m}(CF_2)_m]\text{-}Cl$ | (O) |
| $Cl\text{-}[(CFCl)_{n\text{-}m}(CF_2)_m]\text{-}Cl$ | (P) |

wherein $R_x$, $R_y$, m and n have the meanings given in claim 11.

14. Telomers or mixtures of telomers of 1,2-dichloro-1,2-difluoroethylene according to any one of claims 11 to 13, characterized in that, when $R_x$ and $R_y$ are perhaloalkylpolyether radicals, they contain two oxygen atoms.

15. Telomers or mixtures of telomers of 1,2-dichloro-1,2-difluoroethylene according to any one of claims 11 to 14, characterized in that $R_x$ and $R_y$ are perfluorinated radicals or that, when $R_x$ and $R_y$ contain chlorine atoms, said radicals do not comprise -CCl₃ groups.

16. Telomers or mixtures of telomers of 1,2-dichloro-1,2-difluoroethylene according to any one of claims 11 to 15, characterized in that $R_x$ contains from 1 to 6 and $R_y$ contains from 1 to 5 carbon atoms.